# EUROPEAN PATENT APPLICATION

(11) **EP 3 888 677 A1**
(43) Date of publication of application: **06.10.2021**
(21) Application number: 19884182.7
(22) Date of filing: 14.11.2019
(51) Int. Cl.: A61K 39/275, A61P 31/16, A61K 35/76, C12N 7/01, C12N 15/44

(54) **STRAIN DIS-DERIVED RECOMBINANT VACCINIA VIRUS HAVING NOVEL INFLUENZA VIRUS-DERIVED HEMAGGLUTININ PROTEIN GENE**

(30) Priority: 14.11.2018 JP 2018213990
(71) Applicant: Tokyo Metropolitan Institute of Medical Science, Tokyo 156-8506 (JP); SHIGA UNIVERSITY OF MEDICAL SCIENCE, Otsu-shi Shiga 520-2192 (JP)
(72) Inventor: YASUI, Fumihiko, Tokyo 156-8506 (JP); KOHARA, Michinori, Tokyo 156-8506 (JP); YAMAJI, Kenzaburo, Tokyo 156-8506 (JP); ITOH, Yasushi, Ohtsu-shi, Shiga 520-2192 (JP); OGASAWARA, Kazumasa, Ohtsu-shi, Shiga 520-2192 (JP); ISHII, Koji, Tokyo 162-8640 (JP)
(74) Representative: J A Kemp LLP
(86) International application number: PCT/JP2019/044737
(87) International publication number: WO 2020/100990

(57) **Abstract**

Provided are: a recombinant vaccinia virus which is effective for the prevention of the development of a disease by the infection by H7 avian influenza virus and has high safety; and a vaccine against H7 avian influenza virus, which comprises the recombinant vaccinia virus. The recombinant vaccinia virus according to the present invention is a recombinant vaccinia virus having such a structure that an expression promoter and the full length or a part of cDNA encoding hemagglutinin protein of H7 avian influenza virus are contained in the genome for vaccinia virus strain DIs.

SEQUENCE LISTING

<110>  Tokyo Metropolitan Institute of Medical Science; Shiga
       University of Medical Science;  National Institute of Infectious
       Diseases

<120>  DIs strain-derived recombinant vaccinia virus having novel
       influenza virus-derived hemagglutinin protein gene

<130>  G2076WO

<150>  JP2018-213990
<151>  2018-11-14

<160>  4

<170>  PatentIn version 3.5

<210>  1
<211>  1683
<212>  DNA
<213>  Influenza virus

<400>  1
atgaacactc aaatcctggt attcgctctg attgcgatca ttccaacaaa tgcagacaaa        60

atctgcctcg gacatcatgc cgtgtcaaac ggaaccaaag taaacacatt aactgaaaga       120

ggagtggaag tcgtcaatgc aactgaaaca gtggaacgaa caaacatccc caggatctgc       180

tcaaaaggga aaaggacagt tgacctcggt caatgtggac tcctggggac aatcactgga       240

ccacctcaat gtgaccaatt cctagaattt tcagccgatt taattattga gaggcgagaa       300

ggaagtgatg tctgttatcc tgggaaattc gtgaatgaag aagctctgag gcaaattctc       360

agagaatcag gcggaattga caaggaagca atgggattca catacagtgg aataagaact       420

aatggagcaa ccagtgcatg taggagatca ggatcttcat tctatgcaga aatgaaatgg       480

ctcctgtcaa acacagataa tgctgcattc ccgcagatga ctaagtcata taaaaataca       540

agaaaaagcc cagctctaat agtatggggg atccatcatt ccgtatcaac tgcagagcaa       600

accaagctat atgggagtgg aaacaaactg gtgacagttg ggagttctaa ttatcaacaa       660

tcttttgtac cgagtccagg agcgagacca caagttaatg gtctatctgg aagaattgac       720

tttcattggc taatgctaaa tcccaatgat acagtcactt tcagtttcaa tggggctttc       780

atagctccag accgtgcaag cttcctgaga ggaaaatcta tgggaatcca gagtggagta       840

caggttgatg ccaattgtga aggggactgc tatcatagtg gagggacaat aataagtaac       900

ttgccatttc agaacataga tagcagggca gttggaaaat gtccgagata tgttaagcaa       960

aggagtctgc tgctagcaac agggatgaag aatgttcctg agattccaaa gggaagaggc      1020

ctatttggtg ctatagcggg tttcattgaa aatggatggg aaggcctaat tgatggttgg      1080

tatggtttca gacaccagaa tgcacaggga gagggaactg ctgcagatta caaaagcact      1140

caatcggcaa ttgatcaaat aacaggaaaa ttaaaccggc ttatagaaaa aaccaaccaa      1200

caatttgagt tgatagacaa tgaattcaat gaggtagaga agcaaatcgg taatgtgata 1260

aattggacca gagattctat aacagaagtg tggtcataca atgctgaact cttggtagca 1320

atggagaacc agcatacaat tgatctggct gattcagaaa tggacaaact gtacgaacga 1380

gtgaaaagac agctgagaga gaatgctgaa gaagatggca ctggttgctt tgaaatattt 1440

cacaagtgtg atgatgactg tatggccagt attagaaata acacctatga tcacagcaaa 1500

tacagggaag aggcaatgca aaatagaata cagattgacc cagtcaaact aagcagcggc 1560

tacaaagatg tgatactttg gtttagcttc ggggcatcat gtttcatact tctagccatt 1620

gtaatgggcc ttgtcttcat atgtgtaaag aatggaaaca tgcggtgcac tatttgtata 1680

taa 1683

<210> 2
<211> 1683
<212> DNA
<213> Artificial Sequence

<220>
<223> influenza virus

<400> 2
atggacactc aaatcctggt attcgctctg attgcgatca ttccaacaaa tgcagacaaa 60

atctgcctcg gacatcatgc cgtgtcaaac ggaaccaaag taaacacatt aactgaaaga 120

ggagtggaag tcgtcaatgc aactgaaaca gtggaacgaa caaacatccc caggatctgc 180

tcaaaaggga aaaggacagt tgacctcggt caatgtggac tcctggggac aatcactgga 240

ccacctcaat gtgaccaatt cctagaattt tcagccgatt taattattga gaggcgagaa 300

ggaagtgatg tctgttatcc tgggaaattc gtgaatgaag aagctctgag gcaaattctc 360

agagaatcag gcggaattga caaggaagca atgggattca catacagtgg aataagaact 420

aatggagcaa ccagtgcatg taggagatca ggatcttcat tctatgcaga aatgaaatgg 480

ctcctgtcaa acacagataa tgctgcattc ccgcagatga ctaagtcata taaaaataca 540

agaaaaagcc cagctctaat agtatggggg atccatcatt ccgtatcaac tgcagagcaa 600

accaagctat atgggagtgg aaacaaactg gtgacagttg ggagttctaa ttatcaacaa 660

tcttttgtac cgagtccagg agcgagacca caagttaatg gtctatctgg aagaattgac 720

tttcattggc taatgctaaa tcccaatgat acagtcactt tcagtttcaa tggggctttc 780

atagctccag accgtgcaag cttcctgaga ggaaaatcta tgggaatcca gagtggagta 840

caggttgatg ccaattgtga aggggactgc tatcatagtg gagggacaat aataagtaac 900

ttgccatttc agaacataga tagcagggca gttggaaaat gtccgagata tgttaagcaa 960

aggagtctgc tgctagcaac agggatgaag aatgttcctg agattccaaa gggaagaggc 1020

ctatttggtg ctatagcggg tttcattgaa aatggatggg aaggcctaat tgatggttgg 1080

tatggtttca gacaccagaa tgcacaggga gagggaactg ctgcagatta caaaagcact 1140

caatcggcaa ttgatcaaat aacaggaaaa ttaaaccggc ttatagaaaa aaccaaccaa 1200

caatttgagt tgatagacaa tgaattcaat gaggtagaga agcaaatcgg taatgtgata 1260

aattggacca gagattctat aacagaagtg tggtcataca atgctgaact cttggtagca 1320

atggagaacc agcatacaat tgatctggct gattcagaaa tggacaaact gtacgaacga 1380

gtgaaaagac agctgagaga gaatgctgaa gaagatggca ctggttgctt tgaaatattt 1440

cacaagtgtg atgatgactg tatggccagt attagaaata acacctatga tcacagcaaa 1500

tacagggaag aggcaatgca aaatagaata cagattgacc cagtcaaact aagcagcggc 1560

tacaaagatg tgatactttg gtttagcttc ggggcatcat gtttcatact tctagccatt 1620

gtaatgggcc ttgtcttcat atgtgtaaag aatggaaaca tgcggtgcac tatttgtata 1680

taa 1683

<210> 3
<211> 70
<212> DNA
<213> Vaccinia virus promoter mH5

<400> 3
aaaaattgaa aataaataca aaggttcttg agggttgtgt taaattgaaa gcgagaaata 60

atcataaata 70

<210> 4
<211> 560
<212> PRT
<213> Influenza virus

<400> 4

Met Asn Thr Gln Ile Leu Val Phe Ala Leu Ile Ala Ile Ile Pro Thr
1               5                   10                  15

Asn Ala Asp Lys Ile Cys Leu Gly His His Ala Val Ser Asn Gly Thr
            20                  25                  30

Lys Val Asn Thr Leu Thr Glu Arg Gly Val Glu Val Val Asn Ala Thr
        35                  40                  45

Glu Thr Val Glu Arg Thr Asn Ile Pro Arg Ile Cys Ser Lys Gly Lys
    50                  55                  60

Arg Thr Val Asp Leu Gly Gln Cys Gly Leu Leu Gly Thr Ile Thr Gly
65                  70                  75                  80

Pro Pro Gln Cys Asp Gln Phe Leu Glu Phe Ser Ala Asp Leu Ile Ile
                85                  90                  95

Glu Arg Arg Glu Gly Ser Asp Val Cys Tyr Pro Gly Lys Phe Val Asn
            100                 105                 110

Glu Glu Ala Leu Arg Gln Ile Leu Arg Glu Ser Gly Gly Ile Asp Lys
        115                 120                 125

Glu Ala Met Gly Phe Thr Tyr Ser Gly Ile Arg Thr Asn Gly Ala Thr
    130                 135                 140

Ser Ala Cys Arg Arg Ser Gly Ser Ser Phe Tyr Ala Glu Met Lys Trp
145                 150                 155                 160

Leu Leu Ser Asn Thr Asp Asn Ala Ala Phe Pro Gln Met Thr Lys Ser
                165                 170                 175

Tyr Lys Asn Thr Arg Lys Ser Pro Ala Leu Ile Val Trp Gly Ile His
            180                 185                 190

His Ser Val Ser Thr Ala Glu Gln Thr Lys Leu Tyr Gly Ser Gly Asn
        195                 200                 205

Lys Leu Val Thr Val Gly Ser Ser Asn Tyr Gln Gln Ser Phe Val Pro
    210                 215                 220

Ser Pro Gly Ala Arg Pro Gln Val Asn Gly Leu Ser Gly Arg Ile Asp
225                 230                 235                 240

Phe His Trp Leu Met Leu Asn Pro Asn Asp Thr Val Thr Phe Ser Phe
                245                 250                 255

Asn Gly Ala Phe Ile Ala Pro Asp Arg Ala Ser Phe Leu Arg Gly Lys
            260                 265                 270

Ser Met Gly Ile Gln Ser Gly Val Gln Val Asp Ala Asn Cys Glu Gly
        275                 280                 285

Asp Cys Tyr His Ser Gly Gly Thr Ile Ile Ser Asn Leu Pro Phe Gln
    290                 295                 300

Asn Ile Asp Ser Arg Ala Val Gly Lys Cys Pro Arg Tyr Val Lys Gln
305                 310                 315                 320

Arg Ser Leu Leu Leu Ala Thr Gly Met Lys Asn Val Pro Glu Ile Pro
                325                 330                 335

Lys Gly Arg Gly Leu Phe Gly Ala Ile Ala Gly Phe Ile Glu Asn Gly
            340                 345                 350

Trp Glu Gly Leu Ile Asp Gly Trp Tyr Gly Phe Arg His Gln Asn Ala
        355                 360                 365

Gln Gly Glu Gly Thr Ala Ala Asp Tyr Lys Ser Thr Gln Ser Ala Ile
    370                 375                 380

Asp Gln Ile Thr Gly Lys Leu Asn Arg Leu Ile Glu Lys Thr Asn Gln
385                 390                 395                 400

Gln Phe Glu Leu Ile Asp Asn Glu Phe Asn Glu Val Glu Lys Gln Ile
                405                 410                 415

Gly Asn Val Ile Asn Trp Thr Arg Asp Ser Ile Thr Glu Val Trp Ser
            420                 425                 430

Tyr Asn Ala Glu Leu Leu Val Ala Met Glu Asn Gln His Thr Ile Asp
        435                 440                 445

Leu Ala Asp Ser Glu Met Asp Lys Leu Tyr Glu Arg Val Lys Arg Gln
    450                 455                 460

Leu Arg Glu Asn Ala Glu Glu Asp Gly Thr Gly Cys Phe Glu Ile Phe
465                 470                 475                 480

His Lys Cys Asp Asp Asp Cys Met Ala Ser Ile Arg Asn Asn Thr Tyr
                485                 490                 495

Asp His Ser Lys Tyr Arg Glu Glu Ala Met Gln Asn Arg Ile Gln Ile
            500                 505                 510

Asp Pro Val Lys Leu Ser Ser Gly Tyr Lys Asp Val Ile Leu Trp Phe
        515                 520                 525

Ser Phe Gly Ala Ser Cys Phe Ile Leu Leu Ala Ile Val Met Gly Leu
    530                 535                 540

Val Phe Ile Cys Val Lys Asn Gly Asn Met Arg Cys Thr Ile Cys Ile
545                 550                 555                 560

## Description

### TECHNICAL FIELD

The present invention relates to a prophylactic medicine and therapeutic medicine for H7 subtype avian influenza virus infection that causes the onset of a new type influenza. Specifically, the present invention relates to a recombinant vaccinia virus DIs strain, which can express the hemagglutinin (HA) protein gene of H7 subtype avian influenza virus, and a prophylactic medicine and therapeutic medicine for a new type influenza comprising the recombinant vaccinia virus DIs strain.

### BACKGROUND ART

Seasonal influenza epidemics occur every winter, where 10 to 20% of the population is reported to be affected. The pathogen of such influenza could be influenza virus type A/H1N1, A/H3N2 or B but their antigenicity slightly changes every year and thus a vaccine needs to be produced in accordance with the strain that is predicted to dominate. Meanwhile, there are new types of influenza such as highly pathogenic H5N1 avian influenza, H7N9 avian influenza and highly pathogenic H7N7 avian influenza. In particular, epidemics of infection with H7N9 avian influenza virus in humans have been reported from China since 2013. To date, at least 1564 infected cases and 612 deaths have been reported. Further, the infection scale is the largest in 2016-2017 season when compared to the previous four seasons, and there is concern for further epidemic expansion in future. Currently, in preparation for epidemic expansion of infection with a new type influenza virus in humans, candidate strains for vaccine production have been selected and stored in a plurality of countries including the United States and the United Kingdom. Preparation has been made to immediately start vaccine production in case of emergencies.

However, vaccines planned to be produced are inactivated whole virus particle vaccines, and for this reason, it is required to conduct inoculation with a vaccine a plurality of times, and accordingly, it takes time to obtain sufficient immunity induction. Moreover, it is reported that a sequence that causes activation of suppressor T cells (Treg) is contained in the hemagglutinin (HA) protein sequence of H7N9 avian influenza virus (Non-Patent Document 1), and there is a possibility that the ability to induce an antibody specific to H7 HA protein may be reduced. In consideration of temporal transition of H1N1 (2009) pandemic influenza virus, since the first wave of the epidemic of domestic infection reached each country 2 to 3 months after the epidemic in the country where it occurred first, it is required to develop a vaccine which can respond to H7 subtype influenza virus more quickly.

At present, therapeutic agents for influenza include Tamiflu and Relenza that inhibit the function of neuraminidase (NA) that is essential for the budding of the influenza virus, but their effectiveness is limited because, for example, it is critical to administer them within 48 hours following the onset. Beside them, an intravenous agent of a NA inhibitor has already been developed. Furthermore, although manufacturing and sales of Avigan, an influenza virus polymerase inhibitor, were approved in Japan in March 2014, it can only be manufactured and distributed under certain conditions such as upon a request from the Minister of Health, Labour and Welfare and cannot be sold in the general market.

Among the variety of vaccines, a live vaccine is one of the most effective vaccines. However, the development of an attenuated vaccine for an emerging virus is generally known to take a very long time, and it is considered that the same applies to H7 subtype influenza. The present inventors diligently made researches, and making full use of genetic engineering techniques, developed a recombinant vaccinia virus (RVV) that expresses the H7 subtype HA protein gene as an influenza live vaccine based on a vaccinia virus. So far, recombinant vaccinia viruses (RVV) against rabies virus and rinderpest developed by the present inventors have been demonstrated to exert an excellent effect in preventing infection and onset in the field tests and else (Non-Patent Document 2). In addition, for severe acute respiratory syndrome (SARS), the production of a recombinant vaccinia virus that has cDNA of SARS-CoV known as a pathogen thereof has also been successful (Patent Document 1), and it has been confirmed to be a formulation that shows an excellent preventive effect and that can be repeatedly administered (Non-Patent Document 3). Furthermore, for H5N1 HPAIV, the production of a recombinant vaccinia virus having cDNA of H5N1 HPAIV HA has also been successful, and it has been confirmed to be a formulation that shows an excellent preventive effect (Patent Document 2 and Non-Patent Document 4).

### PRIOR ART DOCUMENTS

### PATENT DOCUMENTS

Patent Document 1: International Publication WO2006/038742 pamphlet
Patent Document 2: Japanese Patent No. 5884100

### NON-PATENT DOCUMENTS

Non-Patent Document 1: Liu R, et al., H7N9 T-cell epitopes that mimic human sequences are less immunogenic and may induce Treg-mediated tolerance. Hum Vaccin Immunother. 2015; 11(9): pp. 2241-52
Non-Patent Document 2: Tsukiyama K. et al., Development of heat-stable recombinant rinderpest vaccine. Arch. Virol., 1989, vol. 107, pp. 225-235
Non-Patent Document 3: Kitabatake M. et al., SARS-CoV spike protein-expressing recombinant vaccinia virus efficiently induces neutralizing antibodies in rabbits pre-immunized with vaccinia virus. Vaccine, 2007, vol. 25, pp. 630-637
Non-Patent Document 4: Yasui F. et al., Sensitization with vaccinia virus encoding H5N1 hemagglutinin restore immune potential against H5N1 influenza virus. Sci. Rep., 2016, vol. 6, p. 37915

### SUMMARY OF THE INVENTION

### PROBLEMS TO BE SOLVED BY THE INVENTION

Under such circumstances, it has been strongly desired to establish a therapeutic medicine and prophylactic medicine comprising a recombinant vaccinia virus (RVV) effective for preventing the onset of a disease caused by H7 subtype avian influenza virus infection.

### MEANS FOR SOLVING THE PROBLEMS

One embodiment of the present invention is as described below.
<1> A recombinant vaccinia virus, wherein an expression promoter and the full length or a part of a cDNA encoding hemagglutinin protein derived from H7 subtype avian influenza virus are contained in the genome of vaccinia virus DIs strain.
<2> The recombinant vaccinia virus according to item <1>, wherein said H7 subtype avian influenza virus is H7N9 avian influenza virus or highly pathogenic H7N7 avian influenza virus.
<3> The recombinant vaccinia virus according to item <1> or <2>, wherein said cDNA encoding hemagglutinin protein is any one of DNAs of (a) to (h) below:
   (a) a DNA consisting of a nucleotide sequence represented by SEQ ID NO: 1;
   (b) a DNA, which hybridizes to a DNA consisting of a nucleotide sequence complementary to the nucleotide sequence represented by SEQ ID NO: 1 under stringent conditions, and which encodes hemagglutinin protein derived from a virus strain belonging to H7N9;
   (c) a DNA, which consists of a nucleotide sequence obtained by deletion, substitution or addition of one or several bases in the nucleotide sequence represented by SEQ ID NO: 1, and which encodes hemagglutinin protein derived from a virus strain belonging to H7N9;
   (d) a DNA, which has at least 75% identity to the nucleotide sequence represented by SEQ ID NO: 1, and which encodes hemagglutinin protein derived from a virus strain belonging to H7N9;
   (e) a DNA consisting of a nucleotide sequence represented by SEQ ID NO: 2;
   (f) a DNA, which hybridizes to a DNA consisting of a nucleotide sequence complementary to the nucleotide sequence represented by SEQ ID NO: 2 under stringent conditions, and which encodes hemagglutinin protein derived from a virus strain belonging to H7N9;
   (g) a DNA, which consists of a nucleotide sequence obtained by deletion, substitution or addition of one or several bases in the nucleotide sequence represented by SEQ ID NO: 2, and which encodes hemagglutinin protein derived from a virus strain belonging to H7N9;
   (h) a DNA, which has at least 75% identity to the nucleotide sequence represented by SEQ ID NO: 2, and which encodes hemagglutinin protein derived from a virus strain belonging to H7N9.
<4> A pharmaceutical composition comprising the recombinant vaccinia virus according to any one of items <1> to <3>.
<5> The pharmaceutical composition according to item <4>, which is a prophylactic medicine for H7 subtype avian influenza.
<6> The pharmaceutical composition according to item <4>, which is a therapeutic medicine for H7 subtype avian influenza.
<7> The pharmaceutical composition according to item <5> or <6>, wherein said H7 subtype avian influenza is H7N9 avian influenza or highly pathogenic H7N7 avian influenza.
<8> The pharmaceutical composition according to any one of items <4> to <7>, which is for intradermal administration.
<9> A formulation for intradermal administration comprising the recombinant vaccinia virus according to any one of items <1> to <3>.
<10> The formulation for intradermal administration according to item <9>, which is a prophylactic medicine for H7 subtype avian influenza.
<11> The formulation for intradermal administration according to item <9>, which is a therapeutic medicine for H7 subtype avian influenza.
<12> The formulation for intradermal administration according to item <10> or <11>, wherein said H7 subtype avian influenza is H7N9 avian influenza or highly pathogenic H7N7 avian influenza.
<13> An influenza vaccine comprising the recombinant vaccinia virus according to any one of items <1> to <3>.
<14> The influenza vaccine according to item <13>, wherein said influenza is H7 subtype avian influenza.
<15> The influenza vaccine according to item <14>, wherein said H7 subtype avian influenza is H7N9 avian influenza or highly pathogenic H7N7 avian influenza.
<16> The influenza vaccine according to any one of items <13> to <15>, which is for intradermal administration.
<17> A method for preventing and/or treating H7 subtype avian influenza, wherein the method comprises administering to a subject an effective amount of the recombinant vaccinia virus according to any one of items <1> to <3>.
<18> The method according to item <17>, wherein said H7 subtype avian influenza is H7N9 avian influenza or highly pathogenic H7N7 avian influenza.
<19> The method according to item <17> or <18>, wherein said administration is intradermal administration.
<20> The method according to any one of items <17> to <19>, wherein the method comprises administering in combination with a vaccine against another subtype influenza virus.
<21> A combination vaccine comprising the recombinant vaccinia virus according to any one of items <1> to <3> and one or more recombinant vaccinia viruses derived from other subtype influenza viruses.
<22> The combination vaccine according to item <21>, wherein said recombinant vaccinia viruses derived from other subtype influenza viruses are rDIs-H1, rDIs-H3 and rDIs-H5.
<23> Use of the recombinant vaccinia virus according to any one of items <1> to <3> in the manufacture of an influenza vaccine.

### ADVANTAGEOUS EFFECT OF THE INVENTION

According to the present invention, it is possible to provide a novel recombinant vaccinia virus, which is effective for preventing or treating a new type influenza and has high safety, and a prophylactic medicine or therapeutic medicine for a new type influenza comprising the same.

### BRIEF DESCRIPTION OF THE DRAWINGS

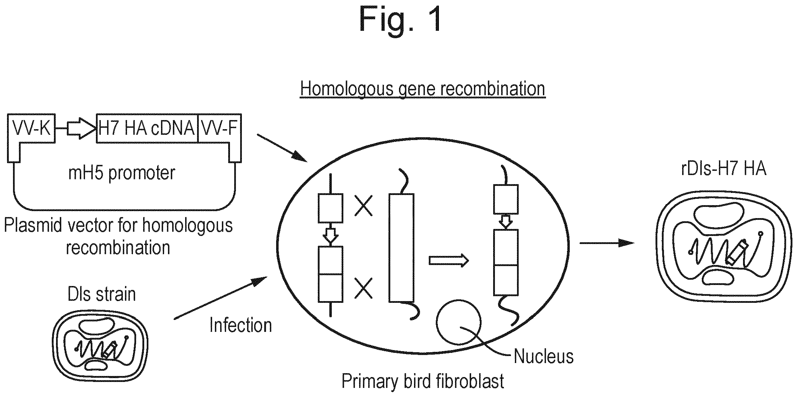

FIG. 1 shows preparation of a DIs strain-derived recombinant vaccinia virus (RVV) having the HA protein gene of H7 subtype avian influenza virus (specifically, rDIs-H7 HA) using the homologous recombination technology.

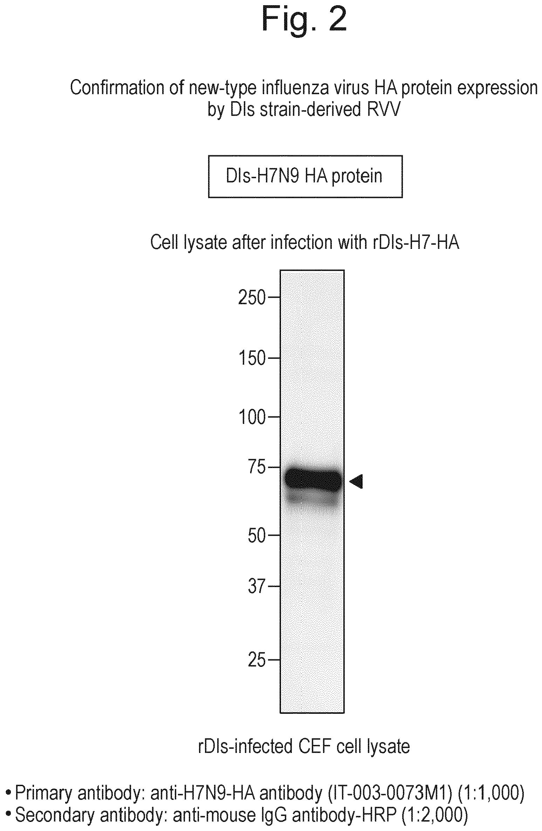

FIG. 2 is a photograph of a PVDF membrane showing results of confirmation of HA protein expression by RVV (rDIs-H7 HA) according to Western blotting.

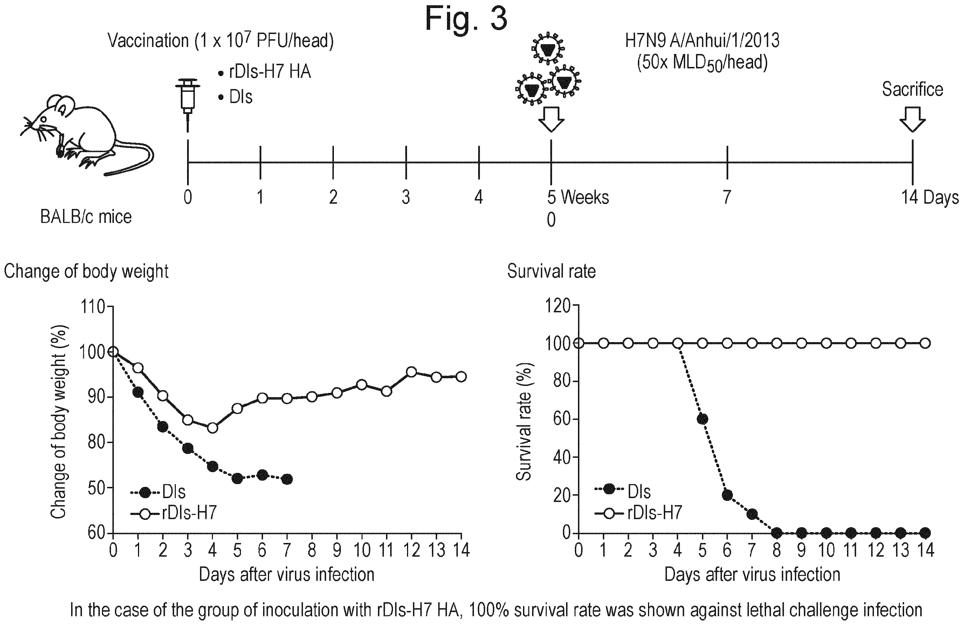

FIG. 3 shows examination results regarding the effect of inducing immune responses and the effect of enhancing the ability to protect against infection in the case of single inoculation with a DIs strain-derived RVV having the HA protein gene derived from H7N9 avian influenza virus (vaccine for H7 subtype avian influenza virus: rDIs-H7 HA) against challenge infection with H7N9 avian influenza virus in BALB/c mice.

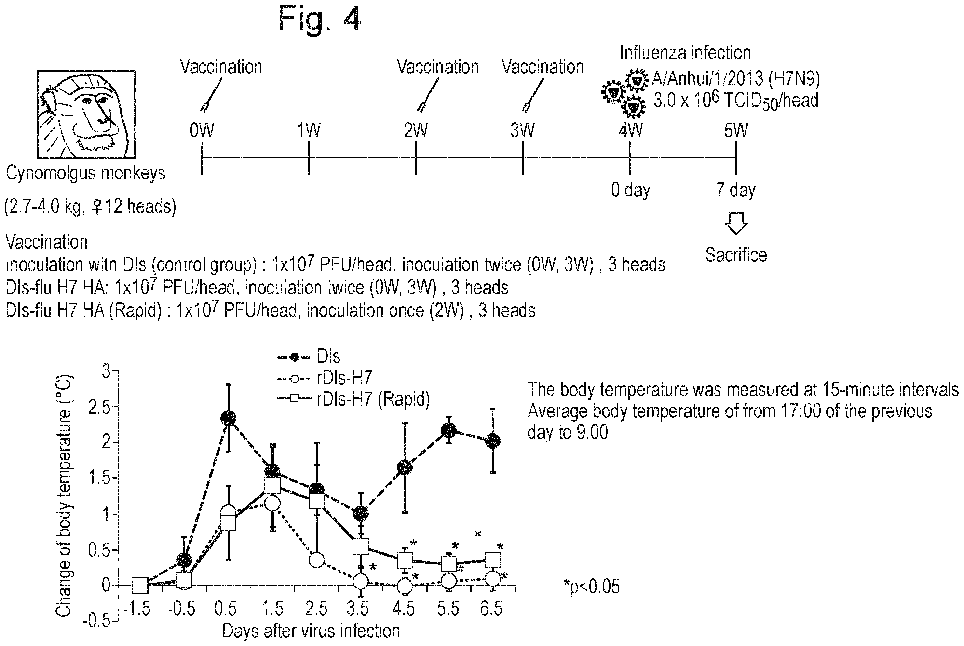

FIG. 4 shows examination results regarding the effect of inducing immune responses, the effect of enhancing the ability to protect against infection and the immediate effect in the case of inoculation with a DIs strain-derived RVV having the HA protein gene derived from H7N9 avian influenza virus (vaccine expressing H7 subtype HA gene: rDIs-H7 HA) using the skin scarification method against challenge infection with H7N9 avian influenza virus in cynomolgus monkeys.

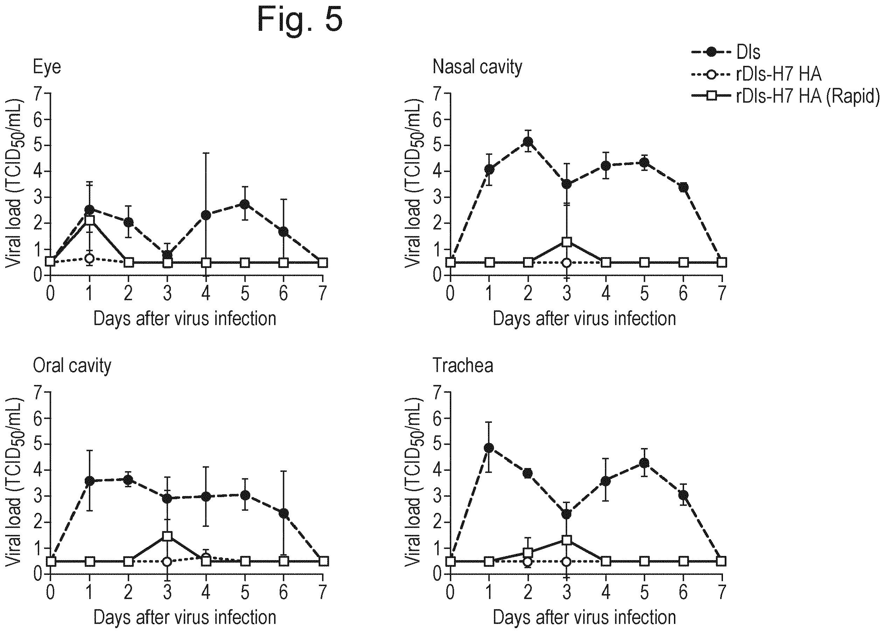

FIG. 5 shows examination results regarding the effect of virus elimination in cynomolgus monkeys inoculated with a vaccine expressing H7 subtype HA gene (rDIs-H7 HA) from the day on which challenge infection with H7N9 avian influenza virus was conducted to 7 days after challenge infection.

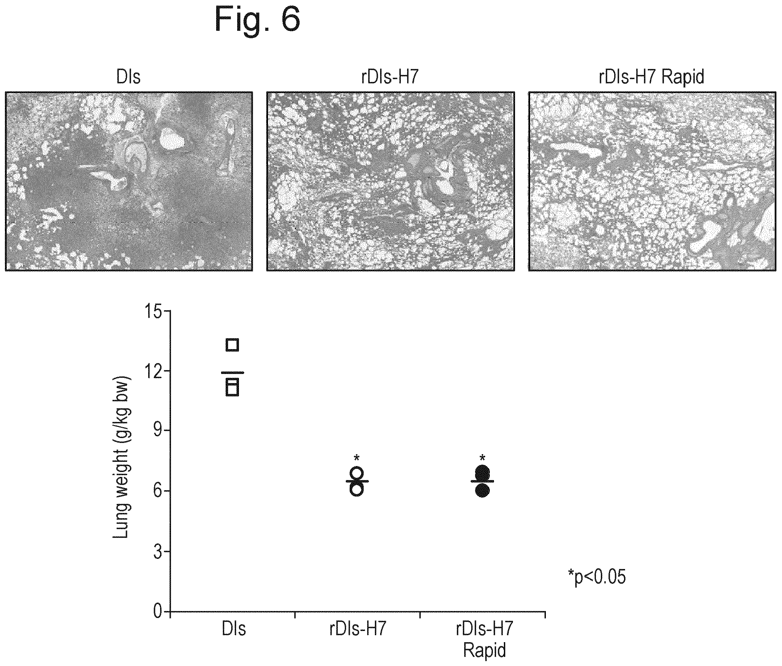

FIG. 6 shows pathological findings of the lung and the lung weight 7 days after challenge infection with H7N9 avian influenza virus in cynomolgus monkeys inoculated with a vaccine expressing H7 subtype HA gene (rDIs-H7 HA).

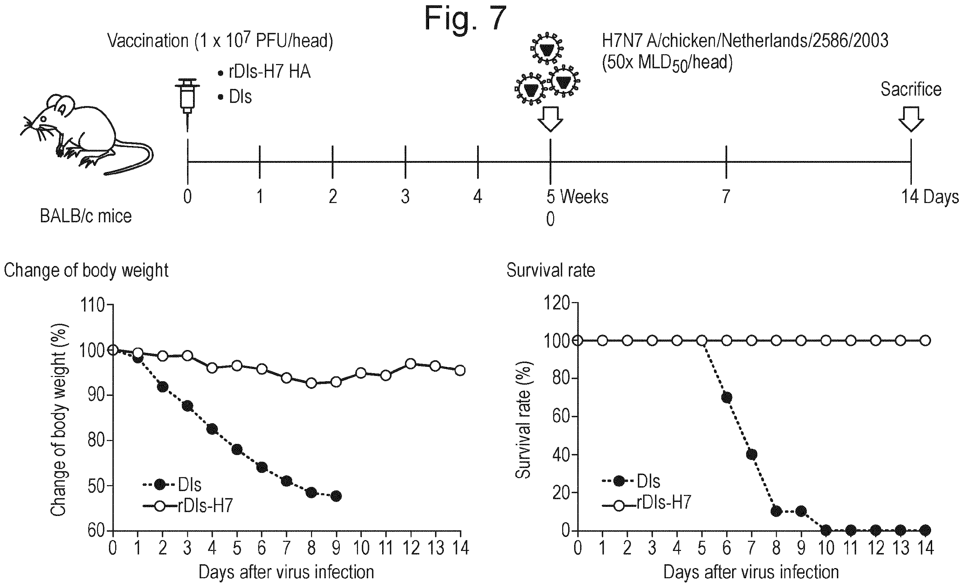

FIG. 7 shows examination results regarding the effect of protection against infection in the case of single inoculation with a DIs strain-derived RVV having the HA protein gene derived from H7N9 (vaccine expressing H7 subtype HA gene: rDIs-H7 HA) against challenge infection with highly pathogenic H7N7 avian influenza virus in BALB/c mice.

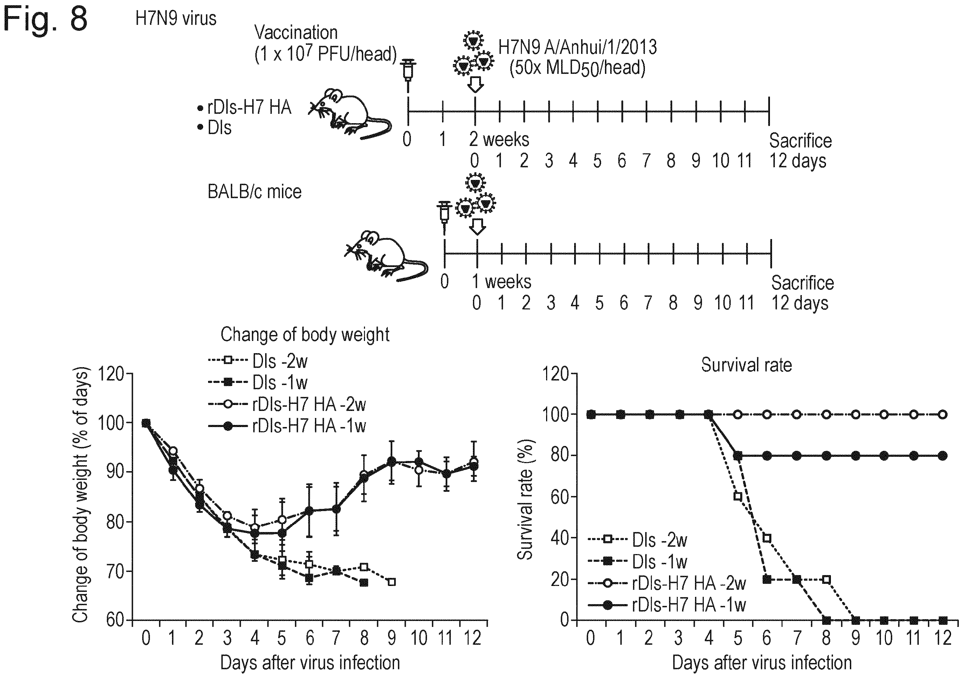

FIG. 8 shows examination results regarding the rapid protection effect of a DIs strain-derived RVV having the HA protein gene derived from H7N9 (vaccine expressing H7 subtype HA gene: rDIs-H7 HA) against challenge infection with H7N9 avian influenza virus in BALB/c mice.

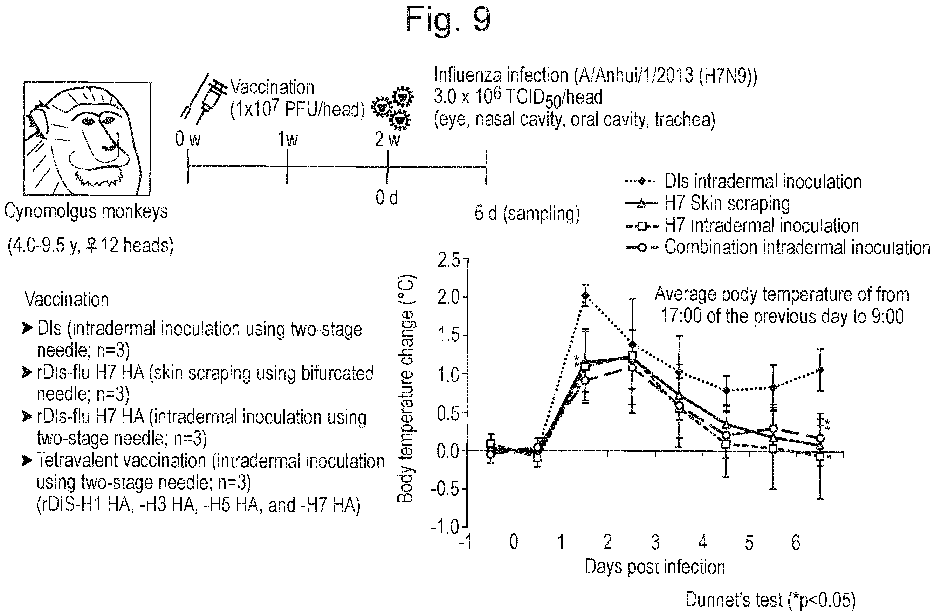

FIG. 9 shows examination results regarding the effect of inducing immune responses and the effect of enhancing the ability to protect against infection in the cases of: inoculation with a monovalent vaccine of a DIs strain-derived RVV having the HA protein gene derived from H7N9 avian influenza virus (vaccine expressing H7 subtype HA gene: rDIs-H7 HA) using the intradermal inoculation method or skin scarification method; and intradermal inoculation with a quadruple vaccine including vaccines against HI, H3 and H5 subtype viruses in addition to H7 influenza virus (rDIs-H1, -H3, -H5 -H7 HAs), each against challenge infection with H7N9 avian influenza virus in cynomolgus monkeys.

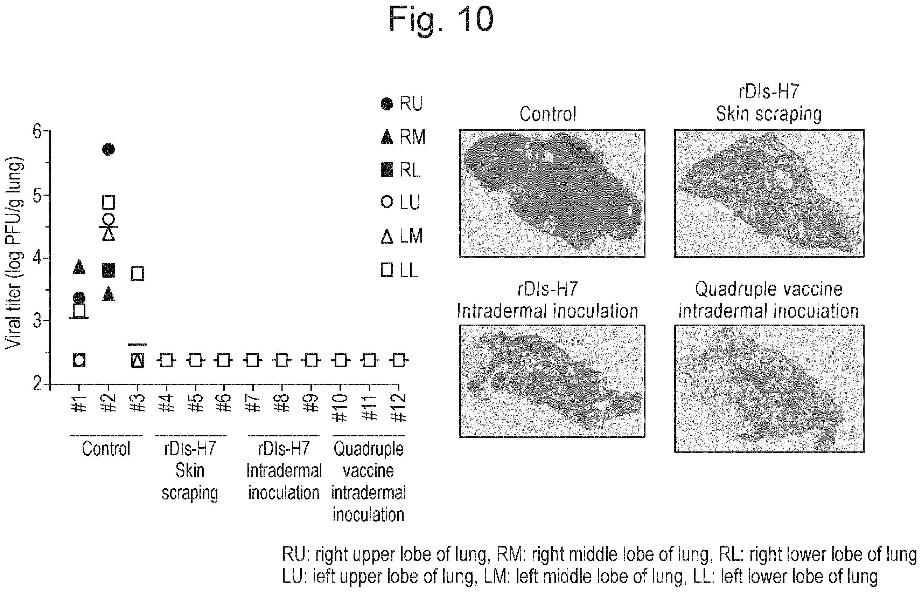

FIG. 10 shows pathological findings of the lung and the viral titer in the lung tissue 7 days after inoculation with a monovalent vaccine of a vaccine expressing H7 subtype HA gene (rDIs-H7 HA) using the intradermal inoculation method or skin scarification method, a quadruple vaccine including vaccines against HI, H3 and H5 subtype viruses in addition to H7 influenza virus (rDIs-H1, -H3, -H5 -H7 HAs) using the intradermal inoculation method or challenge infection with H7N9 avian influenza virus in cynomolgus monkeys.

### MODE FOR CARRYING OUT THE INVENTION

Hereinafter, the recombinant vaccinia virus of the present invention and intended use thereof will be described in detail. The scope of the present invention should not be limited to the following description, and the present invention may suitably be modified and carried out in any way apart from the following illustration without departing from the gist of the present invention. The patent documents, the non-patent documents and other publications cited herein are incorporated herein by reference.

### 1. Outline of the present invention

Under the above-described circumstances, in consideration of vaccination of many and unspecified subjects, the present inventors developed a DIs strain-derived recombinant vaccinia virus (RVV) having an H7 subtype influenza virus-derived hemagglutinin protein gene by using a vaccinia virus DIs strain, which is non-proliferative in mammalian cells and therefore is highly safe, and which has actually been used for vaccinating humans.

As a technique to be employed in such a case, a genetic engineering technique for preparing a recombinant vaccinia virus (RVV) as a live vaccine is known. A vaccinia virus that serves as a recombinant parent for preparing a RVV needs to be a vaccine strain with established safety, and examples of such vaccine strains include vaccinia virus DIs strain and LC16m8 strain. The vaccinia virus DIs strain is a highly attenuated vaccinia virus strain separated by passaging vaccinia virus Dairen strain (DEI strain) in chicken egg embryos (Tagaya I, et al., A new mutant of dermovaccinia virus. Nature, 1961, vol. 192, pp. 1187-1188). This DIs strain can propagate in chicken embryonic fibroblasts (CEF) but is non-proliferative in other mammalian cells and thus is highly safe. Meanwhile, vaccinia virus LC16m8 strain was approved as a smallpox vaccine by the then Ministry of Health and Welfare in 1975, and is a safe and effective attenuated vaccine strain that has actually been used for vaccinating more than 50,000 infants. Attenuation of the LC16m8 strain owes to the fact that the full-length B5R protein is not expressed due to a single base deletion in the B5R protein gene of LC16mO strain that is considered to be involved in adhesion to cells and infection (Morikawa S., et al. An attenuated LC16m8 smallpox vaccine: analysis of full-genome sequence and induction of immune protection. J. Virol., 2005, vol. 79(18), pp. 11873-1189). When using such a vaccine strain with established safety, application to a subject undergoing immunodeficiency or immunosuppression can be realized, and a recombinant live influenza vaccine having advantages such as leaving no vaccine scar can be developed. Therefore, a new type of recombinant influenza vaccine was established based on this DIs strain, and it was aimed to establish a prevention and treatment method for a rapidly spreading new type influenza using a novel vaccine showing effectiveness even in the case of a single inoculation and a short period of time.

As described in detail below, in individuals vaccinated with rDIs-H7 HA, the present invention showed satisfactory effects of protection against H7N9 avian influenza virus infection and H7N7 avian influenza virus infection. Moreover, in the case of infection with H7N9 avian influenza virus 1 week or 2 weeks after vaccination, symptoms were successfully attenuated significantly, and the rapid protection effects of rDIs-H7 HA were confirmed. According to the above-described results, rDIs-H7 HA can serve as a vaccine for H7 subtype influenza which can immediately induce the specific immune responses.

As described above, the present inventors established a new type of recombinant influenza vaccine (rDIs-H7 HA) based on the DIs strain. In mice that had been given a single inoculation with rDIs-H7 HA, temporal weight loss was observed after challenge infection with A/Anhui/1/2013 (H7N9) influenza virus, but then they rapidly gained weight and showed 100% survival rate (Figure 3). In the test of protection against the onset in cynomolgus monkeys, the vaccinated group (inoculation with rDIs-H7 HA twice) showed the effect of more quickly bringing down fever (Figure 4), the effect of earlier virus elimination in swab samples collected from respective parts (conjunctiva, nasal cavity, oral cavity and respiratory tract) (Figure 5) and the effect of remarkable alleviation of pneumonia (Figure 6) when compared to the unvaccinated group (inoculation with DIs). Further, when it was examined whether or not rDIs-H7 HA can respond to rapid spread of infection as a prophylactic vaccine when single inoculation thereof is conducted 2 weeks before infection with H7N9 influenza virus using cynomolgus monkeys, rDIs-H7 HA exerted the rapid protection effect on the onset remarkably even in the case of a short period of time (2 weeks before virus infection) (Figure 4 to Figure 6). The rapid protection effect against the onset was also confirmed in the case of mice (Figure 8). In addition, the mice inoculated with rDIs-H7 HA showed the effect of suppressing weight loss and 100% survival rate even after challenge infection with A/chicken/Netherlands/2586/2003 (H7N7) influenza virus (Figure 7). Thus, when single inoculation with 1 x 10⁷ PFU of the DIs strain-derived RVV expressing the influenza virus HA protein gene, as a vaccine for H7 subtype influenza virus (rDIs-H7 HA), was conducted, even against challenge infection with lethal H7 influenza virus, the effect of suppressing weight loss was shown, and remarkable protective effects on the onset (80% survival rate in the case of inoculation 1 week before, and 100% survival rate in the case of inoculation 2 weeks before) were recognized. Since it is reported that a sequence that causes activation of suppressor T cells (Treg) is contained in the hemagglutinin (HA) protein sequence of H7N9 avian influenza virus (Non-Patent Document 1) and the possibility that the ability to induce an antibody specific to H7 HA protein may be reduced is suggested, there was concern with respect to protective effects of the vaccine, but sufficient effects were recognized as described above. Accordingly, the DIs strain-derived RVV has sufficient effects as a vaccine for H7 subtype influenza virus.

### 2. Preparation of recombinant vaccinia virus having HA protein gene of new influenza virus

A method for preparing a H5N1 HPAIV vaccine is already-known (Japanese Patent No. 5884100), and the vaccine for H7 subtype influenza virus of the present invention (rDIs-H7 HA) can be prepared according to this already-known method. The outline of the preparation method will be described below.

The HA protein gene of H7 subtype avian influenza virus, for example, H7N9 avian influenza virus represented by SEQ ID NO: 1 has already been registered with a predetermined accession number (Accession No. EPI439507) in the influenza virus gene database (GISAID; Global Initiative on Sharing All Influenza Data).

Such HA protein genes (including partially mutated genes) have already been cloned and inserted into plasmids. Therefore, the whole (including mutated sequences) or a part of the gene contained in the recombinant vaccinia virus of the present invention, i.e., cDNA encoding the HA protein of H7 subtype avian influenza virus of H7N9 avian influenza virus can be obtained by a general genetic engineering technique. For example, it is possible to use a nucleic acid synthesis method using a DNA synthesis apparatus, which is generally used as a genetic engineering technique. Further, a PCR method, in which a gene sequence as a template is isolated or synthesized; then a primer specific to each gene is designed; and the gene sequence is amplified using a PCR device, or a gene amplification method using a cloning vector can be used. The above-described methods can be conducted with reference to "Molecular Cloning, A Laboratory Manual (4th edition)" (Cold Spring Harbor Laboratory Press (2012)), etc. For purifying a PCR product obtained, an already-known method can be used. Further, a mutated sequence, in particular, a substitution mutation type DNA can be prepared, for example, in accordance with site-directed mutagenesis described in "Molecular Cloning, A Laboratory Manual (4th edition)" (Cold Spring Harbor Laboratory Press (2012)) described above, "Current Protocols in Molecular Biology, John Wiley & Sons (1987-1997)", etc. Specifically, preparation can be carried out using a mutagenesis kit utilizing site-directed mutagenesis in accordance with an already-known technique such as the Kunkel method and the Gapped duplex method. Preferred examples of the kit include QuickChangeTM Site-Directed Mutagenesis Kit (manufactured by Agilent Technologies), GeneTailorTM Site-Directed Mutagenesis System (manufactured by Invitrogen) and TaKaRa Site-Directed Mutagenesis System (Mutan-K, Mutan-Super Express Km, etc.: manufactured by Takara Bio Inc.).

In a preferred embodiment of the present invention, the HA protein gene of H7 subtype avian influenza virus inserted in the above-described plasmid can be used as the above-described PCR template. In a more preferred embodiment of the present invention, the HA protein gene of H7N9 avian influenza virus can be used as the above-described PCR template. Further, by using a cDNA of the HA protein gene of highly pathogenic H7 subtype avian influenza virus, more preferably H7N9 avian influenza virus as a template and conducting PCR using a primer specific to each gene, the HA protein gene region of highly pathogenic H7 subtype avian influenza virus, more preferably H7N9 avian influenza virus can be prepared.

Each mutant DNA described above can be obtained by chemical synthesis, or can be obtained from a cDNA library and a genome library according to an already-known hybridization method such as colony hybridization, plaque hybridization and Southern blotting, using a DNA consisting of a nucleotide sequence represented by SEQ ID NO: 1 or SEQ ID NO: 2 or a fragment thereof as a probe. Examples of stringent conditions for the above-described hybridization include 0.1×SSC to 10×SSC, 0.1% to 1.0% SDS and 20°C to 80°C. More specifically, for example, prehybridization is performed at 37°C to 56°C for 30 minutes or more, and then washing is performed in 0.1×SSC, 0.1% SDS at room temperature for 10 to 20 minutes from once to three times. For detailed procedures of the hybridization method, "Molecular Cloning, A Laboratory Manual 4th ed." (Cold Spring Harbor Press (2012)), etc. can be referred to.

Further, it is possible to use a DNA (mutant DNA), which has at least 75%, at least 80%, at least 90%, at least 95%, at least 98% or at least 99% homology to the nucleotide sequence represented by SEQ ID NO: 1, and which encodes the HA protein derived from H7 subtype avian influenza virus; and a DNA (mutant DNA), which has at least 75%, at least 80%, at least 90%, at least 95%, at least 98% or at least 99% homology to the nucleotide sequence represented by SEQ ID NO: 2, and which encodes the HA protein derived from H7N9 avian influenza virus.

The expression promoter contained in the recombinant vaccinia virus of the present invention is not limited as long as it can be inserted into a gene deletion region of the vaccinia virus DIs strain, and it may, for example, be vaccinia virus promoter mH5. As said expression promoter, for example, a hybrid promoter consisting of a poxvirus A-type inclusion (ATI) promoter and repeats of a vaccinia virus 7.5 kDa protein (p7.5) early expression promoter may be used. This promoter can be linked to a suitable plasmid. For example, pBMSF7C and pUC/DIs are known (see Arch. Virol. vol. 138, pp. 315-330, 1994; and Japanese Laid-Open Patent Publication No. H06-237773).

The nucleotide sequence of the vaccinia virus promoter mH5 which can be used in the present invention is represented by SEQ ID NO: 3. In the present invention, in addition to a DNA consisting of the nucleotide sequence represented by SEQ ID NO: 3, a DNA, which hybridizes to a DNA consisting of a nucleotide sequence complementary to the nucleotide sequence represented by SEQ ID NO: 3 under stringent conditions, and which has promoter activity, can also be used as the expression promoter (particularly hybrid promoter). The "stringent conditions" are the same as those described above. The expression "has promoter activity" means having transcriptional activity of a gene encoding a structural protein or non-structural protein. A protein expressed by the above-described hybrid promoter can be expressed in a large amount in a form that has undergone complete sugar modification from the early stage to the late stage of vaccinia virus infection.

A plasmid vector obtained by inserting the DNA encoding the expression promoter and the HA protein is introduced into a vaccinia virus as a host, thereby preparing a recombinant vaccinia virus. The plasmid vector can be introduced into the host by employing any known technique. For example, by introducing a plasmid vector obtained into an animal cell infected with an attenuated vaccinia virus DIs strain, homologous recombination is caused in a sequence abutting the gene deletion region of the vaccinia virus DIs strain, and accordingly, a recombinant vaccinia virus (rDIs-H7 HA), by which the HA protein gene of H7 subtype avian influenza virus (e.g., H7N9 avian influenza virus or highly pathogenic H7N7 avian influenza virus) is expressed, can be prepared.

Methods for producing an inactivated vaccine are already known. As mentioned above, an influenza virus used for the vaccine production is propagated by inoculating embryonated chicken eggs with the virus, then the virus purified/concentrated from the chorioallantoic fluid is partially degraded with ether, and the resultant is further inactivated with formalin.

In the prepared DIs strain-derived recombinant vaccinia virus (rDIs-H7 HA), the HA protein gene of H7 subtype avian influenza virus (e.g., H7N9 avian influenza virus) is inserted in the gene deletion region of the vaccinia virus DIs strain. While the influenza virus-derived HA protein is involved in agglutination reaction of guinea pig red blood cells, the vaccinia virus-derived HA protein does not cause agglutination reaction of guinea pig red blood cells. Therefore, RVV is screened by infecting animal cells with rDIs-H7 HA and using agglutination reaction of guinea pig red blood cells to the resulting plaque as an indicator. For intended RVV, red plaques having hemagglutination activity may be selected.

Regarding the virus obtained from the red plaques, PCR is conducted with a primer specific to the HA protein gene of H7 subtype avian influenza virus such as H7N9 avian influenza virus, using the virus genome as a template, and thus introduction of the HA protein gene of H7 subtype avian influenza virus can be confirmed.

The expression of the HA protein of H7 subtype avian influenza virus such as H7N9 avian influenza virus can be confirmed by Western blotting, using an animal cell after infected with rDIs-H7 HA as a sample. As an antibody, a rabbit antiserum prepared by immunization with an HA peptide (SEQ ID NO: 4 or the like) derived from the HA protein of H7N9 avian influenza virus can be used.

As an insertion site of a target gene in the preparation of RVV, the thymidine kinase (TK) gene region is generally used as the gene deletion region of the vaccinia virus DIs strain. However, it is known that proliferation property of RVV is reduced by defective expression of TK due to insertion of a target gene into the TK gene region. Accordingly, in the present invention, as an insertion site of a target gene, the gene deletion region of the vaccinia virus DIs strain is preferred.

### 3. Pharmaceutical composition for preventing and treating new type influenza and use thereof

The present invention provides a prophylactic/therapeutic medicine (pharmaceutical composition for preventing and treating) for a new type influenza (i.e., H7N9 avian influenza and highly pathogenic H7N7 avian influenza), which comprises the above-described recombinant vaccinia virus derived from the DIs strain. Further, the present invention can also provide: a method for preventing and treating the new type influenza, which includes administering the recombinant vaccinia virus to a patient (subject); use of the recombinant vaccinia virus for preventing and treating the new type influenza; use of the recombinant vaccinia virus for producing a prophylactic/therapeutic medicine for the new type influenza; etc.

The pharmaceutical composition of the present invention may be introduced into a living body by any already-known method, for example, an intramuscular, intraperitoneal, intradermal or subcutaneous injection, a nasal, oral or pulmonary inhalation, or an oral administration. Moreover, the recombinant vaccinia virus contained in the pharmaceutical composition of the present invention may be used in combination with an existing antiviral drug (e.g., Tamiflu and Relenza). The mode of combined use is not particularly limited, and the recombinant vaccinia virus of the present invention can be administered simultaneously with the existing antiviral drug. Alternatively, they can be introduced into a living body by administering one of them and then administering the other after a certain lapse of time.

The pharmaceutical composition of the present invention may be mixed with an already-known pharmaceutically acceptable carrier such as an excipient, a filler, a binder or a lubricant, a buffer, a tonicity agent, a chelating agent, a coloring agent, a preservative, a fragrance, a flavoring agent, a sweetener or the like.

The pharmaceutical composition of the present invention can be administered orally or parenterally according to the dosage form, for example, as an oral administration agent such as tablets, capsules, powder, granules, pills, a liquid agent or a syrup, or as a parenteral administration agent such as an injection, a topical medication, a suppository or eye drops. Preferable examples include local injections such as intradermal, intramuscular and intraperitoneal injections. In a preferred embodiment of the present invention, the above-described pharmaceutical composition may be for intradermal administration.

In one embodiment of the present invention, the pharmaceutical composition of the present invention may be provided as a formulation for intradermal administration comprising the recombinant vaccinia virus of the present invention. In one embodiment of the present invention, the formulation for intradermal administration may be a prophylactic medicine for H7 subtype avian influenza. In one embodiment of the present invention, the formulation for intradermal administration may be a therapeutic medicine for H7 subtype avian influenza. In such embodiments, H7 subtype avian influenza may be H7N9 avian influenza or highly pathogenic H7N7 avian influenza.

In another embodiment of the present invention, a method for preventing and/or treating H7 subtype avian influenza, which includes administration of an effective amount of the recombinant vaccinia virus of the present invention to a subject, is provided. In such an embodiment, H7 subtype avian influenza may be H7N9 avian influenza or highly pathogenic H7N7 avian influenza. In a preferred embodiment of the present invention, administration of the recombinant vaccinia virus of the present invention may be carried out by means of intradermal administration.

The dosage may appropriately be selected according to the type of the active component, the administration route, the administration target, the age, weight, sex and symptoms of the patient, and other conditions. The daily dosage of the virus is about 1,000 to 1,000,000,000 PFU (plaque forming units), and preferably about 100,000 to 100,000,000 PFU for oral administration, and about 100 to 1,000,000,000 PFU, and preferably about 1,000 to 100,000,000 PFU for parenteral administration. The virus may be given once or in several times a day.

The recombinant vaccinia virus of the present invention can be used as a vaccine for preventing and treating a new type influenza. Further, in the development of a vaccine against H7 subtype avian influenza virus, researches have been made with attention being focused on an antibody against H7N9 avian influenza virus or highly pathogenic H7N7 avian influenza virus and a cytotoxic T cell (CTL). Accordingly, it is preferred to measure the antibody titer or cell-mediated immune activity as a vaccine in advance. In a preferred embodiment of the present invention, the above-described vaccine for preventing and treating influenza may be for intradermal administration.

For example, the antibody titer against the prepared recombinant vaccinia virus (rDIs-H7 HA) or the DIs strain as the parent strain can be obtained by a procedure in which: a mouse is inoculated with the virus strain; after that, serum is collected over time; and the ELISA value against the HA protein of H7N9 avian influenza virus or highly pathogenic H7N7 avian influenza virus in the serum is measured.

Thus, the recombinant vaccinia virus prepared by the present inventors can induce humoral immunity against a new type influenza.

The recombinant vaccinia virus of the present invention can be used in the production of the influenza vaccine of the present invention.

### 4. Combination vaccine and use thereof

In one embodiment of the present invention, the vaccine comprising the recombinant vaccinia virus of the present invention can be administered in combination with a vaccine against another subtype influenza virus. In one embodiment of the present invention, the above-described administration in combination may be, for example, administering a combination vaccine which comprises the recombinant vaccinia virus of the present invention and a recombinant vaccinia virus derived from another subtype influenza virus.

In one embodiment of the present invention, a combination vaccine comprising the recombinant vaccinia virus of the present invention and one or more recombinant vaccinia viruses derived from other subtype influenza viruses is provided. In one embodiment of the present invention, one or more recombinant vaccinia viruses derived from other subtype influenza viruses may be contained in the combination vaccine of the present invention. In one embodiment of the present invention, the combination vaccine of the present invention may be a double vaccine. In another embodiment of the present invention, the combination vaccine of the present invention may be a triple vaccine. In yet another embodiment of the present invention, the combination vaccine of the present invention may be a quadruple vaccine. In one embodiment of the present invention, the recombinant vaccinia viruses derived from other subtype influenza viruses may be, for example, rDIs-H1, rDIs H3 and rDIs-H5, but are not limited thereto. In a preferred embodiment of the present invention, the combination vaccine of the present invention may be a quadruple vaccine (rDIs-H1, -H3, -H5 -H7 HAs). The combination vaccine of the present invention can be prepared using a method known in the art.

Hereinafter, the present invention will be described more specifically by way of examples. These examples are provided merely for illustration and are not intended to limit the scope of the present invention.

### EXAMPLES

### [Example 1]

### <Method>

### 1) Preparation of DIs strain-derived recombinant vaccinia virus in which influenza virus hemagglutinin protein gene is introduced

A hemagglutinin protein (HA) gene, namely, an antigen of H7N9 influenza virus (A/Anhui/1/2013) artificially synthesized, was linked downstream from a vaccinia virus promoter mH5 sequence, and the resultant was inserted into a plasmid vector for homologous recombination, thereby preparing RVV (Figure 1). For insertion of the H7N9 subtype HA sequence, SphI and AsiSI sites were used as restriction enzyme sites. The prepared plasmid vector for homologous recombination (linearized by cleaving a single site with a restriction enzyme) was transfected into CEF that had been infected with a DIs strain in advance to cause homologous recombination in the region abutting the gene deletion site of the DIs strain, thereby producing a recombinant vaccinia virus (RVV) in which the mH5 promoter and the influenza virus HA protein gene were inserted into the genome of the vaccinia virus, specifically, rDIs-H7 HA. While the influenza virus-derived HA protein is involved in agglutination reaction of guinea pig red blood cells, the vaccinia virus-derived HA protein does not cause agglutination reaction of guinea pig red blood cells. Therefore, RVV was screened by infecting CEF with the above-described recombinant virus and using agglutination reaction of guinea pig red blood cells to the resulting plaque as an indicator. Those that formed red plaques having hemagglutination activity were selected as the recombinant virus of interest.

### 2) Confirmation of influenza virus HA protein expression by DIs strain-derived recombinant vaccinia virus

CEF was infected with a DIs strain-derived recombinant vaccinia virus prepared using SEQ ID NO: 1 (rDIs-H7 HA) with viral infectivity (multiplicity of infection) of 10. 24 hours after the infection, the medium was removed and washing was performed twice using PBS(-). A lysate was added to prepare a cell lysate. SDS-polyacrylamide electrophoresis was carried out with the amount of protein contained in the cell lysate of 10 µg, and transfer to a PVDF membrane was carried out. For confirmation of the influenza virus protein expression by RVV infection, with respect to H7N9 avian influenza virus, a mouse anti-H7N9-HA protein monoclonal antibody was used as the primary antibody, and a sheep anti-mouse IgG antibody-HRP was used as the secondary antibody. A coloring reagent manufactured by Millipore (Millipore; Immobilon western) was used.

### 3) Protective effects against H7N9 avian influenza virus infection and H7N7 avian influenza virus infection in mice given single inoculation with DIs strain-derived recombinant vaccinia virus

BALB/c mice were intradermally inoculated with 1 x 10⁷ PFU of the recombinant vaccinia virus (rDIs-H7 HA) on the back. Challenge infection with H7N9 avian influenza virus (A/Anhui/1/2013) was conducted 5 weeks after the inoculation, and the weight and the survival rate were determined daily. Further, the protective effect on the onset against highly pathogenic H7N7 avian influenza virus (A/chicken/Netherlands/2586/2003), which is also the H7 subtype but a different HA subtype virus, was also examined. In addition, for the purpose of examining the immediate effect of the vaccine, BALB/c mice that were subjected to single inoculation with the recombinant vaccinia virus (rDIs-H7 HA) in the same manner were subjected to challenge infection with H7N9 avian influenza virus (A/Anhui/1/2013) (50x MLD50/individual) 1 week or 2 weeks after the inoculation with the vaccine, and thus the protective effect on the onset was examined.

### 4) Protective effect on onset of recombinant vaccinia virus (rDIs-H7 HA) against H7N9 avian influenza virus infection using cynomolgus monkeys (skin scarification method using bifurcated needle)

Using a bifurcated needle, cynomolgus monkeys were inoculated with 1 x 10⁷ PFU of the recombinant vaccinia virus (rDIs-H7 HA) on both the upper arms (skin scarification method using bifurcated needle). 3 weeks after the initial inoculation, the same amount of the recombinant vaccinia virus (rDIs-H7 HA) was additionally inoculated, and 1 week after the additional inoculation, challenge infection with H7N9 avian influenza virus (A/Anhui/1/2013) (3x10⁶ TCID₅₀/individual) was conducted. In addition, for the purpose of examining the immediate effect of the vaccine, cynomolgus monkeys that were subjected to single inoculation with the recombinant vaccinia virus (rDIs-H7 HA) in the same manner were subjected to challenge infection with H7N9 avian influenza virus (A/Anhui/1/2013) (3x10⁶ TCID₅₀/individual) 2 weeks after the inoculation with the vaccine, and thus the protective effect on the onset was examined.

Using the above-described cynomolgus monkeys, with respect to the unvaccinated group (group of inoculation with DIs), the group of inoculation with the recombinant vaccinia virus (rDIs-H7 HA) twice and the group of inoculation with the recombinant vaccinia virus (rDIs-H7 HA) once (rapid), the body temperature was measured over time from immediately prior to challenge infection, and the lung weight 7 days after the challenge infection was measured.

Further, the collected lung tissue was immersed in a 10% buffered formalin solution to perform virus inactivation and tissue fixation, and after that, a paraffin block of the tissue was prepared. A thin slice of 4 µm was prepared, attached to a slide glass, and then stained with hematoxylin and eosin, and pathological findings of the lung was analyzed.

### <Results>

### i) Protective effects against H7N9 avian influenza virus infection and highly pathogenic H7N7 avian influenza virus infection in mice given single inoculation with DIs strain-derived recombinant vaccinia virus

Although temporal weight loss was observed after the challenge infection with H7N9 influenza virus in the mice that had been given a single inoculation with the prepared RVV (rDIs-H7 HA), they rapidly gained weight and showed 100% survival rate (Figure 3). Meanwhile, in the case of the group of no inoculation with the vaccine (group of inoculation with DIs), rapid weight loss was observed and all the mice died (Figure 3). Further, regarding highly pathogenic H7N7 avian influenza virus infection, the effect of suppressing weight loss and 100% survival rate were shown, and it was confirmed that the vaccine of the present invention is effective not only against H7N9 avian influenza, but also against highly pathogenic H7N7 avian influenza (Figure 7). Moreover, also in the case of the group of single inoculation with the vaccine 1 week or 2 weeks before the challenge infection with H7N9 avian influenza virus, the effect of suppressing weight loss was shown, and 80% survival rate was shown in the case of inoculation 1 week before the challenge infection and 100% survival rate was shown in the case of inoculation 2 weeks before the challenge infection. Thus, remarkable protective effects on the onset were recognized, and it was shown that the vaccine can respond to rapid spread of infection as a prophylactic vaccine (Figure 8).

### ii) Protective effect on onset of recombinant vaccinia virus (rDIs-H7 HA) against H7N9 avian influenza virus infection using cynomolgus monkeys

In the test of the onset protection of the recombinant vaccinia virus (rDIs-H7 HA) against H7N9 avian influenza virus infection using cynomolgus monkeys, a biphasic fever pattern was shown in the case of the unvaccinated group (group of inoculation with DIs), but the effect of quickly bringing down fever was obtained in the case of the vaccinated group with the recombinant vaccinia virus (rDIs-H7 HA) twice (Figure 4). Moreover, also in the case of the group of single inoculation of the recombinant vaccinia virus (rDIs-H7 HA) 2 weeks before the challenge infection in the same manner (rapid), the effect of bringing down fever was similarly confirmed, and the immediate effect of preventing infection was confirmed (Figure 4).

Using the above-described cynomolgus monkeys, with respect to the group of no inoculation with the vaccine (group of inoculation with DIs), the group of inoculation with the recombinant vaccinia virus (rDIs-H7 HA) twice and the group of inoculation with the recombinant vaccinia virus (rDIs-H7 HA) once (rapid), the effect of virus elimination in swab samples collected from the conjunctiva, nasal cavity, oral cavity and respiratory tract was examined. A biphasic pattern was shown in the case of the group of no inoculation with the vaccine (group of inoculation with DIs), but the effect of early virus elimination was confirmed in the case of the group of inoculation with the recombinant vaccinia virus (rDIs-H7 HA) twice and the group of inoculation with the recombinant vaccinia virus (rDIs-H7 HA) once (rapid) (Figure 5).

Using the above-described cynomolgus monkeys, with respect to the group of no inoculation with the vaccine (group of inoculation with DIs), the group of inoculation with the recombinant vaccinia virus (rDIs-H7 HA) twice and the group of inoculation with the recombinant vaccinia virus (rDIs-H7 HA) once (rapid), pathological findings of the lung 7 days after the challenge infection was analyzed. In the case of the group of no inoculation with the vaccine (group of inoculation with DIs), pneumonia was locally recognized. Meanwhile, in the case of both the group of inoculation with the recombinant vaccinia virus (rDIs-H7 HA) twice and the group of inoculation with the recombinant vaccinia virus (rDIs-H7 HA) once (rapid), the alveolar spaces were firmly maintained, and it was shown that pneumonia was remarkably alleviated (Figure 6).

Further, the lung weight was measured. In the case of the group of no inoculation with the vaccine (group of inoculation with DIs), increase in the lung wet weight was recognized. Meanwhile, in the case of both the group of inoculation with the recombinant vaccinia virus (rDIs-H7 HA) twice and the group of inoculation with the recombinant vaccinia virus (rDIs-H7 HA) once (rapid), increase in the lung wet weight was suppressed (Figure 6). Accordingly, it was shown that the vaccine for H7 subtype influenza virus obtained by using the recombinant vaccinia virus of the present invention (rDIs-H7 HA) is effective against H7N9 avian influenza, and that the vaccine can respond to rapid spread of infection as a prophylactic vaccine.

### <Discussion>

Single inoculation with 1 x 10⁷ PFU of the DIs strain-derived RVV expressing the influenza virus HA protein gene as a vaccine for H7 subtype influenza virus (rDIs-H7 HA) showed, against the challenge infection with lethal H7N9 influenza virus and H7N7 influenza virus, the rapid effect of suppressing weight loss and 100% survival rate (administration of the vaccine 2 weeks before the challenge infection). In addition, 80% survival rate was shown even in the case of the administration of the vaccine 1 week before the challenge infection. Note that in the case of the control group (group of inoculation with DIs), all the individuals died. Accordingly, the DIs strain-derived RVV also has sufficient effects, in particular, immediate effects as a vaccine for H7 subtype influenza virus.

### [Example 2]

### Protective effect on onset of recombinant vaccinia virus (rDIs-H7 HA) against H7N9 avian influenza virus infection using cynomolgus monkeys (examination of intradermal inoculation method using two-stage needle)

The method for inoculation with the recombinant vaccinia virus (rDIs-H7 HA) was changed from the skin scarification method to the intradermal inoculation method using a two-stage needle, the protective effect of the vaccine against H7N9 avian influenza virus infection when using the intradermal inoculation method was evaluated in cynomolgus monkeys.

Firstly, inoculation with a monovalent vaccine of the recombinant vaccinia virus (rDIs-H7 HA) using the intradermal inoculation method or skin scarification method (1x10⁷ PFU), or intradermal inoculation with a quadruple vaccine including vaccines against HI, H3 and H5 subtype viruses in addition to H7 influenza virus (rDIs-H1, -H3, -H5 -H7 HAs) (1x10⁷ PFU for each), or intradermal inoculation with DIs (control) were carried out (on both the upper arms of each cynomolgus monkey, single inoculation in each case). 2 weeks after the inoculation, challenge infection with H7N9 avian influenza virus (A/Anhui/1/2013) (3x10⁶ TCID₅₀/individual) was conducted, and the protective effects on the onset were examined.

Using the above-described cynomolgus monkeys, with respect to the group of no inoculation with the vaccine (group of inoculation with DIs; control group), the group of inoculation with the recombinant vaccinia virus (rDIs-H7 HA) by skin scraping, the group of intradermal inoculation with the recombinant vaccinia virus (rDIs-H7 HA) and the group of intradermal inoculation with the quadruple vaccine (rDIs-H1, -H3, -H5 -H7 HAs), the body temperature was measured over time from immediately prior to challenge infection.

As shown in Figure 9, in the case of the group of no inoculation with the vaccine (group of inoculation with DIs; control group), fever was developed immediately after the infection and at the late stage, whereas with respect to the groups of inoculation with the recombinant vaccinia virus (rDIs-H7 HA), in both the cases of the intradermal inoculation method and the skin scraping method, satisfactory suppression of fever and effect of bringing down fever were observed. The results indicate a possibility that the intradermal inoculation method can be used instead of the skin scarification method.

Moreover, also in the case of the group of intradermal inoculation with the quadruple vaccine (rDIs-H1, -H3, -H5 -H7 HAs), suppression of fever and the effect of bringing down fever equivalent to those of the other groups of inoculation with the vaccine were observed. The results indicate a possibility that a vaccine that can simultaneously provide immunities against viruses with 4 serotypes with which humans may be infected can be developed.

Further, regarding the above-described cynomolgus monkeys inoculated or not inoculated with the vaccine, the viral infectivity in the lung tissue was measured and pathological findings of the lung was analyzed. The collected lung tissue was immersed in a 10% buffered formalin solution to perform virus inactivation and tissue fixation, and after that, a paraffin block of the tissue was prepared. A thin slice of 4 µm was prepared, attached to a slide glass, and then stained with hematoxylin and eosin, and pathological findings of the lung was analyzed.

In the case of the group of no inoculation with the vaccine, the infectious virus was detected in a plurality of lobes of the lung and severe pneumonia with pulmonary edema was caused. Meanwhile, in each case of the group of intradermal inoculation with the monovalent vaccine, the group of skin scraping and the group of inoculation with the quadruple vaccine, the titer of infectious virus was equal to or lower than the detection limit, and pneumonia was successfully alleviated remarkably (Figure 10).

According to the above-described results, it was understood that the DIs strain-derived RVV expressing the influenza virus HA protein gene of the present invention serves as a vaccine that is safe and effective against H7 subtype influenza virus. Further, it was shown that the DIs strain-derived RVV expressing the influenza virus HA protein gene of the present invention can respond to rapid spread of infection as a prophylactic vaccine.

### Sequence Listing Free Text

SEQ ID NO: 1: HA protein gene of H7N9 avian influenza virus 
atgaacactcaaatcctggtattcgctctgattgcgatcattccaacaaatgcagacaaaatctgcctcggacatcatgccgtgtc
aaacggaaccaaagtaaacacattaactgaaagaggagtggaagtcgtcaatgcaactgaaacagtggaacgaacaaacat
ccccaggatctgctcaaaagggaaaaggacagttgacctcggtcaatgtggactcctggggacaatcactggaccacctcaa
tgtgaccaattcctagaattttcagccgatttaattattgagaggcgagaaggaagtgatgtctgttatcctgggaaattcgtgaat
gaagaagctctgaggcaaattctcagagaatcaggcggaattgacaaggaagcaatgggattcacatacagtggaataagaa
ctaatggagcaaccagtgcatgtaggagatcaggatcttcattctatgcagaaatgaaatggctcctgtcaaacacagataatg
ctgcattcccgcagatgactaagtcatataaaaatacaagaaaaagcccagctctaatagtatgggggatccatcattccgtatc
aactgcagagcaaaccaagctatatgggagtggaaacaaactggtgacagttgggagttctaattatcaacaatcttttgtaccg
agtccaggagcgagaccacaagttaatggtctatctggaagaattgactttcattggctaatgctaaatcccaatgatacagtca
ctttcagtttcaatggggctttcatagctccagaccgtgcaagcttcctgagaggaaaatctatgggaatccagagtggagtaca
ggttgatgccaattgtgaaggggactgctatcatagtggagggacaataataagtaacttgccatttcagaacatagatagcag
ggcagttggaaaatgtccgagatatgttaagcaaaggagtctgctgctagcaacagggatgaagaatgttcctgagattccaa
agggaagaggcctatttggtgctatagcgggtttcattgaaaatggatgggaaggcctaattgatggttggtatggtttcagaca
ccagaatgcacagggagagggaactgctgcagattacaaaagcactcaatcggcaattgatcaaataacaggaaaattaaac
cggcttatagaaaaaaccaaccaacaatttgagttgatagacaatgaattcaatgaggtagagaagcaaatcggtaatgtgata
aattggaccagagattctataacagaagtgtggtcatacaatgctgaactcttggtagcaatggagaaccagcatacaattgatc
 
tggctgattcagaaatggacaaactgtacgaacgagtgaaaagacagctgagagagaatgctgaagaagatggcactggttg
ctttgaaatatttcacaagtgtgatgatgactgtatggccagtattagaaataacacctatgatcacagcaaatacagggaagag
gcaatgcaaaatagaatacagattgacccagtcaaactaagcagcggctacaaagatgtgatactttggtttagcttcggggca
tcatgtttcatacttctagccattgtaatgggccttgtcttcatatgtgtaaagaatggaaacatgcggtgcactatttgtatataa
SEQ ID NO: 2: Single base substitution type of HA protein gene of H7N9 avian influenza virus 
atggacactcaaatcctggtattcgctctgattgcgatcattccaacaaatgcagacaaaatctgcctcggacatcatgccgtgtc
aaacggaaccaaagtaaacacattaactgaaagaggagtggaagtcgtcaatgcaactgaaacagtggaacgaacaaacat
ccccaggatctgctcaaaagggaaaaggacagttgacctcggtcaatgtggactcctggggacaatcactggaccacctcaa
tgtgaccaattcctagaattttcagccgatttaattattgagaggcgagaaggaagtgatgtctgttatcctgggaaattcgtgaat
gaagaagctctgaggcaaattctcagagaatcaggcggaattgacaaggaagcaatgggattcacatacagtggaataagaa
ctaatggagcaaccagtgcatgtaggagatcaggatcttcattctatgcagaaatgaaatggctcctgtcaaacacagataatg
ctgcattcccgcagatgactaagtcatataaaaatacaagaaaaagcccagctctaatagtatgggggatccatcattccgtatc
aactgcagagcaaaccaagctatatgggagtggaaacaaactggtgacagttgggagttctaattatcaacaatcttttgtaccg
agtccaggagcgagaccacaagttaatggtctatctggaagaattgactttcattggctaatgctaaatcccaatgatacagtca
ctttcagtttcaatggggctttcatagctccagaccgtgcaagcttcctgagaggaaaatctatgggaatccagagtggagtaca
ggttgatgccaattgtgaaggggactgctatcatagtggagggacaataataagtaacttgccatttcagaacatagatagcag
ggcagttggaaaatgtccgagatatgttaagcaaaggagtctgctgctagcaacagggatgaagaatgttcctgagattccaa
agggaagaggcctatttggtgctatagcgggtttcattgaaaatggatgggaaggcctaattgatggttggtatggtttcagaca
ccagaatgcacagggagagggaactgctgcagattacaaaagcactcaatcggcaattgatcaaataacaggaaaattaaac
cggcttatagaaaaaaccaaccaacaatttgagttgatagacaatgaattcaatgaggtagagaagcaaatcggtaatgtgata
aattggaccagagattctataacagaagtgtggtcatacaatgctgaactcttggtagcaatggagaaccagcatacaattgatc
tggctgattcagaaatggacaaactgtacgaacgagtgaaaagacagctgagagagaatgctgaagaagatggcactggttg
ctttgaaatatttcacaagtgtgatgatgactgtatggccagtattagaaataacacctatgatcacagcaaatacagggaagag
gcaatgcaaaatagaatacagattgacccagtcaaactaagcagcggctacaaagatgtgatactttggtttagcttcggggca
tcatgtttcatacttctagccattgtaatgggccttgtcttcatatgtgtaaagaatggaaacatgcggtgcactatttgtatataa
SEQ ID NO: 3: Sequence of vaccinia virus promoter mH5 
aaaaattgaa aataaataca aaggttcttg agggttgtgt taaattgaaa gcgagaaata 60
atcataaata 70
SEQ ID NO: 4: Full-length amino acid sequence of HA of H7N9 avian influenza virus

MNTQILVFALIAIIPTNADKICLGHHAVSNGTKVNTLTERGVEVVNATETVERTNI
PRICSKGKRTVDLGQCGLLGTITGPPQCDQFLEFSADLIIERREGSDVCYPGKFV
NEEALRQILRESGGIDKEAMGFTYSGIRTNGATSACRRSGSSFYAEMKWLLSNT

DNAAFPQMTKSYKNTRKSPALIVWGIHHSVSTAEQTKLYGSGNKLVTVGSSNY
QQSFVPSPGARPQVNGLSGRIDFHWLMLNPNDTVTFSFNGAFIAPDRASFLRGK
SMGIQSGVQVDANCEGDCYHSGGTIISNLPFQNIDSRAVGKCPRYVKQRSLLLAT
GMKNVPEIPKGRGLFGAIAGFIENGWEGLIDGWYGFRHQNAQGEGTAADYKST
QSAIDQITGKLNRLIEKTNQQFELIDNEFNEVEKQIGNVINWTRDSITEVWSYNA
ELLVAMENQHTIDLADSEMDKLYERVKRQLRENAEEDGTGCFEIFHKCDDDCM
ASIRNNTYDHSKYREEAMQNRIQIDPVKLSSGYKDVILWFSFGASCFILLAIVMG
LVFICVKNGNMRCTICI

## Claims

1. A recombinant vaccinia virus, wherein an expression promoter and the full length or a part of a cDNA encoding hemagglutinin protein derived from H7 subtype avian influenza virus are contained in the genome of vaccinia virus DIs strain.

2. The recombinant vaccinia virus according to claim 1, wherein said H7 subtype avian influenza virus is H7N9 avian influenza virus or highly pathogenic H7N7 avian influenza virus.

3. The recombinant vaccinia virus according to claim 1 or 2, wherein said cDNA encoding hemagglutinin protein is any one of DNAs of (a) to (h) below:
(a) a DNA consisting of a nucleotide sequence represented by SEQ ID NO: 1;
(b) a DNA, which hybridizes to a DNA consisting of a nucleotide sequence complementary to the nucleotide sequence represented by SEQ ID NO: 1 under stringent conditions, and which encodes hemagglutinin protein derived from a virus strain belonging to H7N9;
(c) a DNA, which consists of a nucleotide sequence obtained by deletion, substitution or addition of one or several bases in the nucleotide sequence represented by SEQ ID NO: 1, and which encodes hemagglutinin protein derived from a virus strain belonging to H7N9;
(d) a DNA, which has at least 75% identity to the nucleotide sequence represented by SEQ ID NO: 1, and which encodes hemagglutinin protein derived from a virus strain belonging to H7N9;
(e) a DNA consisting of a nucleotide sequence represented by SEQ ID NO: 2;
(f) a DNA, which hybridizes to a DNA consisting of a nucleotide sequence complementary to the nucleotide sequence represented by SEQ ID NO: 2 under stringent conditions, and which encodes hemagglutinin protein derived from a virus strain belonging to H7N9;
(g) a DNA, which consists of a nucleotide sequence obtained by deletion, substitution or addition of one or several bases in the nucleotide sequence represented by SEQ ID NO: 2, and which encodes hemagglutinin protein derived from a virus strain belonging to H7N9;
(h) a DNA, which has at least 75% identity to the nucleotide sequence represented by SEQ ID NO: 2, and which encodes hemagglutinin protein derived from a virus strain belonging to H7N9.

4. A pharmaceutical composition comprising the recombinant vaccinia virus according to any one of claims 1 to 3.

5. The pharmaceutical composition according to claim 4, which is a prophylactic medicine for H7 subtype avian influenza.

6. The pharmaceutical composition according to claim 4, which is a therapeutic medicine for H7 subtype avian influenza.

7. The pharmaceutical composition according to claim 5 or 6, wherein said H7 subtype avian influenza is H7N9 avian influenza or highly pathogenic H7N7 avian influenza.

8. The pharmaceutical composition according to any one of claims 4 to 7, which is for intradermal administration.

9. A formulation for intradermal administration comprising the recombinant vaccinia virus according to any one of claims 1 to 3.

10. The formulation for intradermal administration according to claim 9, which is a prophylactic medicine for H7 subtype avian influenza.

11. The formulation for intradermal administration according to claim 9, which is a therapeutic medicine for H7 subtype avian influenza.

12. The formulation for intradermal administration according to claim 10 or 11, wherein said H7 subtype avian influenza is H7N9 avian influenza or highly pathogenic H7N7 avian influenza.

13. An influenza vaccine comprising the recombinant vaccinia virus according to any one of claims 1 to 3.

14. The influenza vaccine according to claim 13, wherein said influenza is H7 subtype avian influenza.

15. The influenza vaccine according to claim 14, wherein said H7 subtype avian influenza is H7N9 avian influenza or highly pathogenic H7N7 avian influenza.

16. The influenza vaccine according to any one of claims 13 to 15, which is for intradermal administration.

17. A method for preventing and/or treating H7 subtype avian influenza, wherein the method comprises administering to a subject an effective amount of the recombinant vaccinia virus according to any one of claims 1 to 3.

18. The method according to claim 17, wherein said H7 subtype avian influenza is H7N9 avian influenza or highly pathogenic H7N7 avian influenza.

19. The method according to claim 17 or 18, wherein said administration is intradermal administration.

20. The method according to any one of claims 17 to 19, wherein the method comprises administering in combination with a vaccine against another subtype influenza virus.

21. A combination vaccine comprising the recombinant vaccinia virus according to any one of claims 1 to 3 and one or more recombinant vaccinia viruses derived from other subtype influenza viruses.

22. The combination vaccine according to claim 21, wherein said recombinant vaccinia viruses derived from other subtype influenza viruses are rDIs-H1, rDIs-H3 and rDIs-H5.

23. Use of the recombinant vaccinia virus according to any one of claims 1 to 3 in the manufacture of an influenza vaccine.
